# EUROPEAN PATENT APPLICATION

(11) **EP 2 127 655 A1**
(43) Date of publication of application: **02.12.2009**
(21) Application number: 09168219.5
(22) Date of filing: 24.03.2005
(51) Int. Cl.: A61K 31/56, A61P 27/04

(54) **Use of loteprednol etabonate for the treatment of dry eye**

(30) Priority: 25.03.2004 US 556161 P
(62) Divisional of application: 05729286.4
(71) Applicant: BAUSCH & LOMB INCORPORATED, Rochester, New York 14604-2701 (US)
(72) Inventor: Bartels, Stephen P., Pittsford, NY 14534 (US)
(74) Representative: Glas, Holger

(57) **Abstract**

Disclosed in embodiments herein is a method of treating chronic dry eye in a patient in need thereof, the method comprising topically administering to the patient Loteprednol etabonate in an ophthalmolically acceptable carrier.

## Description

### CROSS REFERENCE TO RELATED APPLICATIONS

This application claims the benefit of Provisional Patent Application No. 60/556,161 filed March 25, 2004 and is incorporated herein by reference.

### BACKGROUND AND SUMMARY

Dry eye, also known generically as keratoconjunctivitis sicca (KCS), is a common ophthalmological disorder affecting millions of Americans each year. The condition is particularly widespread among post-menopausal women due to hormonal changes following the cessation of fertilifiy. Dry eye may afflict an individual with varying severity. In mild cases, a patient may experience burning, a feeling of dryness, and persistent irritation such as is often caused by small bodies lodging between the eye lid and the eye surface. In severe cases, vision may be substantially impaired. Other diseases, such as Sjogren's disease manifest dry eye complications.

The human ocular surface is normally covered by a tear film that is composed of a superficial thin lipid layer (primarily derived from meibomian gland secretions), a middle bulk aqueous layer (consisting of proteins, electrolytes, and water secreted by lacrimal glands), and the innermost mucus layer derived from mucins secreted by the ocular surface epithelial cells.

A stable tear film ensures comfort and serves as the refractive optical surface of the eye. Furthermore, the tear film serves as a barrier for the ocular surface against microbial infection and inflammation from mechanical trauma. Tear film deficiencies, referred to as dry eye, are a common clinical problem that can result from decreased secretion of tears by the lacrimal gland and/or increased evaporative loss due to a deficient lipid layer or blink abnormalities. Patients with mild dry eye complain of annoying eye irritations. Those with severe dry eye, such as Sjogren's syndrome, may experience constant and disabling eye irritation, and develop ocular surface epithelial disease and sight-threatening sterile or microbial corneal ulceration.

Corticosteroids are potent, non-specific anti-inflammatory drugs that inhibit a variety of chembtactic substances and factors that mediate capillary permeability, contraction of nonvascular smooth muscle, and vasodilatation. In addition, corticosteroids suppress inflammation by inhibiting edema, fibrin deposition, migration of leukocytes and phagocytic activity.

Topical corticosteroids are useful in a variety of ophthalmic conditions and are generally indicated for treatment of steroid-responsive inflammatory conditions of the palpebral and bulbar conjunctiva, cornea and anterior segment of the eye. Although corticosteroids are widely used as a topical agent for ocular inflammation, most possess a safety risk profile that limits their more general utility. A common risk associated with corticosteroid therapy is an elevation of intraocular pressure (IOP). In addition, chronic use of topical corticosteroids may result in the development of cataracts. Loteprednol etabonate is a compound designed as a site-active corticosteroid that will undergo a predictable transformation to an inactive metabolite. The relatively rapid metabolism of Loteprednol etabonate to an inactive metabolite improves the safety profile of this corticosteroid. This characteristic of Lotemax® (Loteprednol etabonate ophthalmic suspension, 0.5%) makes it an excellent candidate for use in inflammatory ocular conditions.

For years it has been recognized that patients with dry eye develop pathologic changes of the ocular surface epithelial cells termed squamous metaplasia. Unreported research suggests that this process is the result of increased proliferation, abnormal differentiation, and inflammation of the ocular surface epithelial cells. In contrast to normal cells, these metaplastic cells do not produce the mucus that normally coats the ocular surface and forms a barrier against infection and mechanical trauma. This renders the ocular surface susceptible to damage from the mild trauma of desiccation blinking, rubbing, and foreign bodies (such as contact lenses).

Lotemax® (Loteprednol etabonate ophthalmic suspension, 0.5%) was approved by the FDA in March, 1998 for use in the treatment of steroid responsive conditions. Researchers have concluded that there is an inflammatory etiology in some, if not most, cases of dry eye. Reports of clinical studies have supported the use of topical corticosteroid solutions in the treatment of patients with keratoconjunctivitis sicca. Stephen Pflugfelder, MD, introduced the diagnostic test of fluorescein tear clearance to further differentiate and select patients with dry eye with an inflammatory component. It is postulated that the reduced flow of tears through the tear film and out of the cul-de-sac into the naso-lacrimal duct results in stasis, which allows inflammatory mediators in the tear film to remain in contact with the mucosa of the conjunctiva and the corneal epithelium. Stasis, thus, may increase the inflammation associated with the KCS. This process may also be responsible for a further reduction of tear production in such patients.

Basic and clinical research performed over the past decade indicates that inflammation develops on the ocular surface as tear production and tear clearance decrease ^{1,2}. Immunopathological changes have been observed in the conjunctiva of patients with dry eye including increased expression of immune activation and cellular adhesion molecules (e.g. HLA-DR antigen and ICAM-1) as well as infiltration of the epithelium and stroma by inflammatory cells ^{3,4}. Increased levels of pro-inflammatory cytokines such as, IL-1, IL-6 and TNF-α, have been detected in the tear fluid and conjunctival epithelium of patients with dry eye ^{2,5,7,8}. The tear fluid concentration of IL-1a was found to increase as tear clearance decreased². Significantly increased concentration and activity of matrix metalloproteinase-9 (MMP-9) has been detected in the tear fluid of dry eye patients with delayed tear clearance ². Production of MMP-9 by corneal epithelial cells and leukocytes is stimulated by the pro-inflammatory cytokines IL-1 and TNF-α ⁸. Experimental evidence suggests that MMP-9 is involved in the pathogenesis of keratoconjunctivitis sicca by dissolving the tight junction proteins that anchor the differentiated mucin-bearing apical corneal epithelial cells ⁹. Glucocorticosteroids are recognized for their ability to decrease the production of pro-inflammatory cytokines¹⁰ and matrix metalloproteinase enzymes¹¹. Several recent clinical studies have supported the use of topical corticosteroid solutions in the treatment of patients with keratoconjunctivitis sicca ^{12,13,14}

The following recent patents or publications are noted:
US Patent No. 6,153,607 discloses a preservative-free formulation containing an effective amount of a corticosteroid in an aqueous carrier to treat a dry eye condition. The formulation may be provided as a part of a therapeutic regimen to treat a variety of dry eye conditions and ocular surface disorders manifesting delayed tear clearance previously not readily treatable. The formulation may be packaged as containers of single dosage amounts of the corticosteroid-aqueous formulation sufficient for pulsed-therapy of acute exacerbations of the irritation symptoms and ocular surface disease of conditions associated with dry eye and delayed tear clearance.
US Patent Application. Publication No. 20030008853 discloses 22,29-epoxy-3,4,6,7,29-pentahydroxy-,(3α,4β,5α,6α,7- β,14β,22S)-stigmastan-15-one as useful for treating dry eye disorders and other disorders requiring the wetting of the eye. This publication further notes that corticosteroids, such as prednisolone, dexamethasone, fluoromethalone, hydrocortisone, loteprednol, triamcinolone, etc., cannot be used for prolonged therapy in dry eye patients without causing side effects. This publication further notes that steroid-related complications including increased intraocular pressure and cataract formation have been observed in dry eye patients treated with corticosteroids after several months of therapy. Therefore, it was surprisingly discovered that a topical ophthalmic formulation containing Loteprednol etabonate, when used for four weeks, was efficacious and well tolerated in the management of patients with KCS with inflammation. Moreover, a differential treatment effect, which was in some cases significant, was seen in subsets of patients who presented a moderate to severe inflammatory component corresponding with more severe KCS.

Therefore, disclosed in embodiments herein is a method of treating moderate to severe dry eye in patients who present a moderate to severe inflammatory component. Symptoms of severe dry eye may include, amongst others, conjunctival injection (hyperemia); such as bulbar conjunctival hyperemia, inferior tarsal conjunctival hyperemia, nasal bulbar conjunctival hyperemia; lid margin hyperemia, central corneal staining and redness of the eye. The method comprises topically administering to the patient with moderate to severe dry eye Loteprednol etabonate in an ophthalmologically acceptable carrier.

### BRIEF DESCRIPTION OF THE FIGURES

Figure 1 is a graphical representation of the change in mean worse symptom scores for an intent to treat (ITT) analysis. VAS = visual analog score.
Figure 2 is a graphical representation of the mean percentage change from baseline to week 2 intent to treat (ITT) analysis.
Figure 3 is a graphical representation of the mean percentage change from baseline to week 4 intent to treat (ITT) analysis.
Figure 4 is a graphical representation of the mean percentage change from baseline to week 2 in a subset of patients with a corneal staining score >10 at baseline.
Figure 5 is a graphical representation of the mean percentage change from baseline to week 4 in a subset of patients with a corneal staining score >10 at baseline.
Figure 6 is a graphical representation of the mean percentage change from baseline to week 2 in a subset of patients with a conjunctival hyperemia score >2 in any area at baseline.
Figure 7 is a graphical representation of the mean percentage change from baseline to week 4 in patients with a conjunctival hyperemia score >2 in any area at baseline.
Figure 8 is a graphical representation of the mean percentage change from baseline to week 2 in a subset pf patients with a corneal staining score >10 and a conjunctival hyperemia score >2 in any area at baseline.
Figure 9 is a graphical representation of the mean percentage change from baseline to week 4 in a subset of patients with a corneal staining score >10 and a conjunctival hyperemia score >2 in any area at baseline.

### DETAILED DESCRIPTION

Topical steroids for treating ocular inflammations can be based on predictably metabolized drugs. Predictably metabolized drugs, as is known in the art, are designed to provide maximal therapeutic effect and minimal side effects. By one approach, synthesis of a "predictably metabolized drug" can be achieved by structurally modifying a known inactive metabolite of a known active drug to produce an active metabolite that undergoes a predictable one-step transformation in-vivo back to the parent, inactive metabolite (see, U.S. Pat. Nos. 6,610,675, 4,996,335 and 4,710,495 for predictably metabolized steroids). "Predictably metabolized drugs" therefore are biologically active chemical components characterized by predictable in-vivo metabolism to non-toxic derivatives after they provide their therapeutic effect. Formulations of steroids suitable for ophthalmic use are known. For example, US patents 4,710,495, 4,996,335, 5,540,930, 5,747,061, 5,916,550, 6,368,616 and 6,610,675, the contents of each of which is incorporated by reference herein, describe predictably metabolized steroids and/or formulations containing predictably metabolized steroids.

(11β,17α),-17-[(Ethoxycarbonyl)oxy]-11-hydroxy-3-oxoandrosta-1,4-diene-17-carboxylic acid chloromethyl ester (Loteprednol etabonate) is a known compound and can be synthesized by methods disclosed in U.S. Pat. No. 4,996,335, the entire contents of which are hereby incorporated by reference in the present specification.

According to the methods of the present invention, a formulation comprising (11β,17α),-17-[(Ethoxycarbonyl)oxy]-11-hydroxy-3-oxoandrosta-1,4-diene-17-carboxylic acid chloromethyl ester and a pharmaceutically acceptable carrier for topical ophthalmic administration or implantation into the conjunctival sac or anterior chamber of the eye is administered to a mammal in need thereof. The formulations are formulated in accordance with methods known in the art for the particular route of administration desired.

The formulations administered according to the present invention comprise a pharmaceutically effective amount of (11β,17α),-17-[(Ethoxycarbonyl)oxy]-11-hydroxy-3-oxoandrosta-1,4-diene-17-carboxylic acid chloromethyl ester. As used herein, a "pharmaceutically effective amount" is one which is sufficient to reduce or eliminate signs or symptoms of dry eye. Generally, for formulations intended to be administered topically to the eye in the form of eye drops or eye ointments, the amount of (11β,17α),-17-[(Ethoxycarbonyl)oxy]-11-hydroxy-3-oxoandrosta-1,4-diene-17-carboxylic acid chloromethyl ester will be about 0.001 to 5.0% (W/W). For preferred topically administrable ophthalmic formulations, the amount of (11β,17α),-17-[(Ethoxycarbonyl)oxy]-11-hydroxy-3-oxoandrosta-1,4-diene-17-carboxylic acid chloromethyl ester will be about 0.001 to 1.0% (W/W).

The formulations administered according to the present invention may also include various other ingredients, including but not limited to surfactants, tonicity agents, buffers, preservatives, co-solvents and viscosity building agents.

Surfactants that can be used are surface-active agents that are acceptable for ophthalmic or otolaryngological uses. Useful surface active agents include but are not limited to polysorbate 80, tyloxapol, TWEEN 80 (ICI America Inc., Wilmington, Del.), PLURONIC F-68 (from BASF, Ludwigshafen, Germany) and the poloxamer surfactants can also be used. These surfactants are nonionic alkaline oxide condensates of an organic compound which contains hydroxyl groups. The concentration in which the surface active agent may be used is only limited by neutralization of the bactericidal effects on the accompanying preservatives (if present), or by concentrations which may cause irritation.

Various tonicity agents may be employed to adjust the tonicity of the formulation. For example, sodium chloride, potassium chloride, magnesium chloride, calcium chloride, nonionic diols, preferably glycerol, dextrose and/or mannitol may be added to the formulation to approximate physiological tonicity. Such an amount of tonicity agent will vary, depending on the particular agent to be added. In general, however, the formulations will have a tonicity agent in an amount sufficient to cause the final formulation to have an ophthalmically acceptable osmolality (generally about 150-450 mOsm).

An appropriate buffer system (e.g., sodium phosphate, sodium acetate, sodium citrate, sodium borate or boric acid) may be added to the formulations to prevent pH drift under storage conditions. The particular concentration will vary, depending on the agent employed.

Topical ophthalmic products are typically packaged in multidose form. Preservatives are thus required to prevent microbial contamination during use. Suitable preservatives include: benzalkonium chloride, chlorobutanol, benzododecinium bromide, methyl paraben, propyl paraben, phenylethyl alcohol, edetate disodium, sorbic acid, polyquaternium-1, or other agents known to those skilled in the art. Such preservatives are typically employed at a level of from 0.001 to 1.0% W/W. Unit dose formulations of the present invention will be sterile, but typically unpreserved. Such formulations, therefore, generally will not contain preservatives.

Co-solvents and viscosity building agents may be added to the formulations to improve the characteristics of the formulations. Such materials can include nonionic water-soluble polymer. Other compounds designed to lubricate, "wet," approximate the consistency of endogenous tears, aid in natural tear build-up, or otherwise provide temporary relief of dry eye symptoms and conditions upon ocular administration the eye are known in the art. Such compounds may enhance the viscosity of the formulation, and include, but are not limited to: monomeric polyols, such as, glycerol, propylene glycol, ethylene glycol; polymeric polyols, such as, polyethylene glycol, hydroxypropylmethyl cellulose ("HPMC"), carboxy methylcellulose sodium, hydroxy propylcellulose ("HPC"), dextrans, such as, dextran 70; water soluble proteins, such as gelatin; and vinyl polymers, such as, polyvinyl alcohol, polyvinylpyrrolidone, povidone and carbomers, such as, carbomer 934P, carbomer 941, carbomer 940, carbomer 974P. Other compounds may also be added to the ophthalmic formulations of the present invention to increase the viscosity of the carrier. Examples of viscosity enhancing agents include, but are not limited to: polysaccharides, such as hyaluronic acid and its salts, chondroitin sulfate and its salts, dextrans, various polymers of the cellulose family; vinyl polymers; and acrylic acid polymers.

Formulations formulated for the treatment of dry eye-type diseases and disorders may also comprise aqueous carriers designed to provide immediate, short-term relief of dry eye-type conditions. Such carriers can be formulated as a phospholipid carrier or an artificial tears carrier, or mixtures of both. As used herein, "phospholipid carrier" and "artificial tears carrier" refer to aqueous formulations which: (i) comprise one or more phospholipids (in the case of phospholipid carriers) or other compounds, which lubricate, "wet," approximate the consistency of endogenous tears, aid in natural tear build-up, or otherwise provide temporary relief of dry eye symptoms and conditions upon ocular administration; (ii) are safe; and (iii) provide the appropriate delivery vehicle for the topical administration of an effective amount of (11β,17α),-17-[(Ethoxycarbonyl)oxy]-11-hydroxy-3-oxoandrosta-1,4-diene-17-carboxylic acid chloromethyl ester. Examples of artificial tears formulations useful as artificial tears carriers include, but are not limited to, commercial products, such as Moisture Eyes^{™} Lubricant Eye Drops/Artificial Tears, Moisture Eyes^{™} Liquid Gel lubricant eye drops, Moisture Eyes^{™} Preservative Free Lubricant Eye Drops/Artificial Tears and Moisture Eyes™ Liquid Gel Preservative Free Lubricant Eye Drops/Artificial Tears (Bausch & Lomb Incorporated, Rochester, NY). Examples of phospholipid carrier formulations include those disclosed in U.S. Pat. Nos. 4,804,539 (Guo et al.), U.S. Pat. No. 4,883,658 (Holly), U.S. Pat. No. 4,914,088 (Glonek), U.S. Pat. No. 5,075,104 (Gressel et al.), U.S. Pat. No. 5,278,151 (Korb et al.), U.S. Pat. No. 5,294,607 (Glonek et al.), U.S. Pat. No. 5,371,108 (Korb et al.), U.S. Pat. No. 5,578,586 (Glonek et al.), the contents of each of which are incorporated by reference herein.

Formulations formulated for the treatment of dry eye-type diseases and disorders may be prepared as ophthalmic ointments. Ointments are well known ophthalmic formulations and are essentially an oil-based delivery vehicle. Typical ointments use petroleum and/or lanolin base to which is added the active ingredient, usually as 0.1 to 2%, and excipients. Common bases include mineral oil, petrolatum and combinations thereof, but oil bases are not limited thereto.

The preferred formulations of the present invention are intended for administration to a human patient suffering from dry eye or symptoms of dry eye. Preferably, such formulations will be administered topically. In general, the doses used for the above described purposes will vary, but will be in an effective amount to eliminate or improve dry eye conditions. Generally, 1-2 drops of such formulations will be administered from once to many times per day. The formulation is intended to be provided as a package for the treatment of dry eye, the package would include the pharmaceutical formulation comprising Loteprednol etabonate contained in a pharmaceutically acceptable container; a written package insert containing instructions for using the formulation for the treatment of dry eye; and outer packaging identifying the pharmaceutical formulation contained therein. In certain embodiments wherein the formulation is preservative free, the package would contain a pharmaceutically acceptable container suitable for single use by a user of the packaged formulation. In such embodiments it is envisioned that the outer packaging would contain at least one pharmaceutically acceptable container containing the Loteprednol etabonate formulation. Preferably the outer packing would contain a multiplicity of single use containers, for example, enough single use containers to provide for a one-month supply of the formulation.

A clinical trial compared the efficacy of the site-specific steroid loteprednol etabonate with its vehicle in the treatment of keratoconjunctivitis sicca (KCS) in patients with delayed tear clearance. A number of subjective and objective parameters were evaluated in this study. In the intent-to-treat population, loteprednol and vehicle both improved symptoms of eye irritation to a similar extent. After two and four weeks of therapy, the loteprednol treated group showed greater improvement in objective signs than the vehicle group, with this difference reaching statistical significance for inferior tarsal and nasal bulbar conjunctival hyperemia at two weeks and for nasal bulbar hyperemia at four weeks

Clinical experience and several epidemiological studies indicate that dry eye is more prevalent in women ^{17,18}. As expected, the majority (76%) of the enrolled patients in this trial were female. There were a greater percentage of women in the vehicle group than in the Lotemax® group. Patients were not stratified by sex when they were randomly assigned to a treatment group. No treatment effect between males and females was observed and there is no evidence to suggest that there should be a clinical sex related differences in the response of KCS to corticosteroids.

Greater improvement in several objective variables, including central corneal fluorescein staining, nasal bulbar conjunctival hyperemia and symptoms of eye redness were observed in a subset of patients who had more severe clinical inflammatory signs at entry and who were treated with loteprednol compared to those treated with vehicle. Central corneal fluorescein staining improved approximately 30% at two weeks and 20% at four weeks in this subset of patients. This clinical finding was associated with a significant improvement in the videokeratoscopic surface regularity index (SRI) in those patients who were evaluated with this technique. Although not wishing to be bound by a particular theory, applicant believes these findings to be clinically relevant because central corneal staining has been found to be positively correlated with the SRI, as well as with potential visual acuity ¹⁶. Indeed, patients with severe central corneal fluorescein staining often complain of blurred and fluctuating visual acuity.

Conjunctival redness was found to be a steroid-responsive parameter in this cohort of patients with delayed tear clearance. While redness has not been traditionally recognized as a symptom of dry eye, it is a common complaint of patients with delayed tear clearance and it is often most severe in the morning upon awakening ¹⁹. It is not surprising that patients with tear film dysfunction do not often report redness of the non-exposed inferior tarsal conjunctiva since it is not recognized unless they pull their lower lid down and view it in a mirror.

The clinical improvement observed with topical steroid therapy in this relatively small group of dry eye patients supports a role for inflammation in the pathogenesis of keratoconjunctivitis sicca. Potential mechanisms by which corticosteroids may improve ocular surface disease in dry eye were not evaluated in this clinical trial, but previously reported studies have found that steroids decrease the production of inflammatory cytokines by the conjunctival and corneal epithelium as well as the production of matrix metalloproteinase-9 (MMP-9) by the cornea ^{6,10,11}. The levels of these factors could be directly assessed in future trials to determine if their decrease corresponds to an improvement in keratoconjunctivitis sicca.

The more potent steroid methylprednisolone in a nonpreserved formulation was used in previous clinical studies that reported an improvement in keratoconjunctivitis sicca following corticosteroid therapy ^{13,14,19}. While a greater clinical effect was observed in these trials compared to the current study, methylprednisolone carries significant risks of ocular toxicity including ocular hypertension and cataract formation following prolonged use. An impressive finding in our trial of loteprednol was the complete lack of toxicity following one month of therapy. In particular there was neither elevation of the mean IOP nor any individual with a clinically important elevation of the IOP. This suggests that short-term therapy of dry eye for up to one month with this steroid agent carries an excellent benefit-to-risk ratio. The safety of Ioteprenol is further supported by long term follow-up of 265 patients who received 0.2% loteprenol etabonate for up to 3 years for treatment of allergic conjunctivitis.²⁰ Compared to pretreatment values, mean intraocular pressure was not different from baseline after 3 years of treatment. Furthermore, no cataract formation was observed.

The invention will now be further described by way of several examples that are intended to describe but not limit the scope of the invention as defined by the claims herein.
Representative eye drop formulations are provided in Examples 1-4 below.

### EXAMPLE 1

| | Lotemax® (loteprednol etabonate |
|---|---|
| Ingredients (per mL) | ophthalmic suspension, 0.5%) |
| Loteprednol etabonate, NDS, | 5.00 mg |
| micronized, sterile | |
| Povidone, USP(C-30) | 6.00 mg |
| Benzalkonium chloride, 50% | 0.20 mg |
| solution NF | |
| Edetate disodium dihydrate, USP | 0.10 mg |
| Glycerin, USP, 96% | 25.00* mg |
| Tyloxapol, USP | 3.00 mg |
| Purified water, USP | QS to 1 mL |
| Hydrochloric acid, 37%, NF | Adjust pH |
| (diluted to 0.1 N) | |
| Sodium hydroxide, NF (diluted to | Adjust pH |
| 0.1N) | |

| | |
|---|---|
| *25.0 mg/mL of glycerin, 96% is equivalent to 24 mg/mL (2.4 W/W) of glycerin, 100% | |

### EXAMPLE 2

| Ingredient | Amount |
|---|---|
| Phase I | |
| Carbopol 934P NF | 0.25 gm |
| (Acrylic acid-based polymer) | |
| | |
| Purified Water | 99.75 gm |
| | |
| Phase II | |
| Propylene Glycol | 5.0 gm |
| EDTA | 0.1 mg |
| Loteprednol Etabonate | 50.0 gm |

| | |
|---|---|
| Mix five parts of phase II with twenty parts of phase I for more than 15 minute and adjust pH to 6.2-6.4 using 1 N NaOH. | |

### EXAMPLE 3

| Ingredient | Amount |
|---|---|
| Phase I | |
| Carbopol 934P NF | 0.25 gm |
| (Acrylic acid-based polymer) | |
| | |
| Purified Water | 99.75 gm |
| | |
| Phase II | |
| Propylene Glycol | 3.0 gm |
| Triacetin | 7.0 gm |
| Loteprednol Etabonate | 50.0 gm |
| EDTA | 0.1 gm |

| | |
|---|---|
| Mix five parts of phase II with twenty parts of phase I for more than 15 minutes and adjust pH to 6.2-6.4 using 1 N NaOH. | |

### EXAMPLE 4

| Ingredient | Amount |
|---|---|
| Phase I | |
| Carbopol 934P NF | 0.25 gm |
| (Acrylic acid-based polymer) | |
| | |
| Purified Water | 99.75 gm |
| | |
| Phase II | |
| Propylene Glycol | 7.0 gm |
| Glycerin | 3.0 mg |
| Loteprednol Etabonate | 50.0 gm |
| EDTA | 0.1 mg |
| BAK | 01 - 0.2 mg |

Mix five parts of phase II with twenty parts of phase I for more than 15 minutes and adjust pH to 6.2-6.4 using 1 N NaOH.

### PATIENTS AND METHODS

A randomized, double-masked, placebo-controlled, multicenter, pilot study was conducted in compliance with ICH Good Clinical Practice and the Declaration of Helsinki. Approval was obtained from an Institutional Review Board for each study site before initiating the study. Written informed consent was obtained from all patients before any study specific investigations were performed.

### Patient Selection

Eligible patients were 18 years of age or older and had a diagnosis of keratoconjunctivitis sicca (KCS) of at least 6 months duration. In addition, they were required to meet the following criteria in at least one eye:
1. delayed tear clearance defined by a standardized visual scale score ≥ 3 ¹⁵
2. at least one symptom >30mm on the visual analog scale (VAS)
3. composite corneal staining score >3

Females were either postmenopausal or using a recognized, reliable method of contraception and pregnant or lactating females were disqualified. Patients with contraindications to the use of topical corticosteroid eye drops or their components were excluded. Patients with illnesses that could interfere with the study, ocular infections, or a history of herpes simplex infection were also excluded. Further exclusions were made for contact lens use, punctal occlusion within three months, systemic steroid use in the last six months or topical steroid use in the last two months, and for concurrent use of ophthalmic medications for conditions other than KCS. Ophthalmic surgery in the past six months, or participation in another clinical study/use of experimental medication in the past 30 days also disqualified patients from participation.

### Drug Administration

Patients were randomly assigned to receive either Lotemax® Bausch and Lomb Inc. Rochester NY, or placebo four times a day. Lotemax® (loteprednol etabonate ophthalmic suspension, 0.5%) was supplied in 7.5 mL (5.0 mL fill) white, low density polyethylene dropper bottles with white caps for masking purposes. Placebo (the vehicle of Lotemax®) was supplied in identical bottles. Preservative Free Moisture Eyes™ Lubricant Eye Drops (Bausch & Lomb) were supplied in unit dose containers.

Patients were instructed to use their study medication in both eyes four times daily and to use only the Preservative Free Moisture Eyes™ Lubricant Eye Drops provided if symptomatic relief was required. Patients were informed that the lubricant drops were to be the only rescue medication, and were not to be instilled until 10 minutes after the study drug was instilled in order to avoid washing out the study drug. The number of single dose units that patients used would be recorded at each visit.

### Study procedure.

The study procedures are shown in Table 1 below. Patients attended for 5 visits. At Visit 1 patients signed an informed consent and underwent screening examinations (VAS symptom scoring; fluorescein tear clearance ¹³ corneal staining; biomicroscopy; lens exam and fundoscopy and pregnancy test, if applicable). The fluorescein tear clearance test was conducted by observing and grading the fluorescence persisting in the tear film 15 minutes after instillation of 5 microliters of a 2% solution of fluorescein. The fluorescence was graded between 0 and 6 using a standard color comparison scale. Only those patients with an eye with a score of >3 were eligible. The corneal staining was evaluated 5 minutes after the instillation of the 2% fluorescein, viewing the cornea through a slit lamp with blue light and a No. 11 Wratten filter (See Table 2 below).

**TABLE 1. Time and Events Schedule**

| Procedure | Visit 1 | Visit 2 | Visit 3 | Visit 4 | Visit 5 |
|---|---|---|---|---|---|
| | Day-14 to -7 | Day 1 | Day 14±3 | Day 28±3 | Day 42±3 |
| | Start of Run-in | Baseline | | End of Study Drug Period | Off-treatment Follow-up |
| Obtain informed consent, | | | | | |
| conduct pregnancy test | X | | | | |
| Dispense rescue | | | | | |
| medication | X | X | X | X | |
| | | | | | |
| Instill study drug | | X | X | | |
| | | | | | |
| Score VAS symptoms | X | X | X | X | X |
| Perform corneal staining, | | | | | |
| biomicroscopy, VA and IOP | | | | | |
| tests | X | X | X | X | X |
| | | | | | |
| Perform tear clearance test | X | X | | | |
| Perform Schirmer 1 test | | X | | X | X |
| Perform lens exam, | | | | | |
| fundoscopy | X | | | X | |
| Record use of rescue | | | | | |
| medication | | X | X | X | X |
| VA indicates visual acuity; IOP, intraocular pressure | | | | | |

**TABLE 2. Corneal staining grading system**

| | |
|---|---|
| Grade | No Staining |
| 0 | |
| Grade | Less than or equal to 5 areas of punctuate staining |
| 1 | |
| Grade | Greater than 5 but less than 15 areas of punctuate staining OR |
| 2 | one area of coalesced staining |
| Grade | Greater than 15 areas of punctuate staining OR two or more areas |
| 3 | of coalesced staining OR any area of epithelial or stromal diffusion of fluorescein. |
| Grade | Greater than 15 areas of punctuate staining AND two or more |
| 4 | areas of coalesced staining AND a frank corneal epithelial defect. |

| | |
|---|---|
| NB: The corneal staining score was to be graded exactly 5 minutes after instillation of 5µl of a 2% solution of fluorescein, viewed through a slit lamp microscope using a blue light and a No. 11 Wratten filter. | |

Those who met the selection criteria were given a supply of Preservative Free Moisture Eyes™ Lubricant Eye Drops unit dose containers and instructed to use only those drops as needed until their next visit at the end of the 7-day run-in period. At Visit 2, which was regarded as the baseline visit, patients were once again evaluated (fluorescein tear clearance, corneal staining, Schirmer 1 test, and biomicroscopy) and their symptomatology was recorded. Eligible patients received a study number and received double-masked study medication (either Lotemax® or placebo) according to a predetermined random allocation schedule. They were also given a further supply of Preservative Free Moisture Eyes™ Lubricant Eye Drops.

Visits 3 and 4 occurred at weeks 2 and 4 of the treatment period. At these visits corneal staining and biomicroscopy were performed, VAS symptom severity was scored, and use of Moisture Eyes™ was recorded (Schirmer 1 testing was recorded at Visit 4). Study medication was discontinued following visit 4, but an additional supply of rescue medication (Moisture Eyes^{™}) was provided at that visit. Visit 5 took place two weeks after completion of the treatment period. Corneal staining, biomicroscopy, and Schirmer 1 testing were conducted, VAS symptom severity was scored and use of Moisture Eyes™ was recorded at that visit (Table 1 above).

### Clinical Assessment

As is traditional for studies of this kind, two primary variables were defined representing subjective evaluation by the patient and objective evaluation based on the clinical examination findings. The combined corneal staining score, chosen as the primary objective variable, was the sum of the scores for the five areas of the cornea (central, inferior, nasal, superior and temporal). Each area was scored according to the corneal staining grading scale (Table 2 above). A visual analog scale (VAS) score for the worst symptom at Visit 2 was chosen as the subjective variable. The patients were asked to grade the severity of the following symptoms: burning/stinging, itching, grittiness/scratchiness/foreign body sensation, dryness, stickiness, redness of the eye, and tired eye sensation. Symptoms were graded for each eye separately and for the conditions upon waking and during the day. The visual analog scale-a 100mm line, marked at the left end "Absent" and at the right end "Unbearable"-was provided for this purpose. The patient was asked to mark the line at the point between these two extremes that represented the severity of the symptom, the distance from the left end of the line to the mark was the VAS score in mm.

The primary analysis was based on change from Visit 2 to Visit 4 in the visual analog scale score for the worst symptom in the worst eye (at baseline Visit 2), and in the composite fluorescein staining score in the worst eye (at baseline Visit 2). Secondary analyses included the following, calculated as a change from Visit 2 to Visits 4 and 5, respectively: all symptom VAS scores, amount of Preservative Free Moisture Eyes™ Lubricant Eye Drops used, Schirmer 1 test scores, and fluorescein staining scores for the five areas of the cornea. Biomicroscopic findings (lid margin and conjunctival injection, filamentary keratitis) were also assessed.

Safety was assessed by fundoscopy, lens examination and biomicroscopy, by tests of visual acuity and intraocular pressure, and by monitoring adverse events and changes in symptomatology. Adverse events were evaluated by the investigator as to severity (mild, moderate, severe) and relationship of the event to the study drug (probable, possible, unlikely, or unknown).

### Videokeratoscopy:

Videokeratoscopic examination with the TMS-2 Corneal Topography System was performed at one of the study centers using the technique previously reported.¹⁶ Subjects were instructed to view the flashing red fixation light. An image was captured after proper alignment and focusing. The SRI (surface regularity index), SAI (surface asymmetry index), irregular astigmatism index (IAI) and PVA (potential visual acuity index) were calculated by the instrument's software and were recorded in a database (Excel, Microsoft, Seattle, WA).

Demographic and baseline characteristics were summarized by descriptive statistics for all randomized patients. The treatment groups were compared using an analysis of variance (ANOVA). A level of significance of α = 0.05 was employed in all statistical tests of hypotheses.

Since this was a pilot study the calculation of the sample size was based on the best available relevant publications. The present study used similar subjective and objective endpoints or variables evaluating the efficacy of steroids for treatment of KCS that were used in a previously reported study by Sainz de la Maza Serra et al.,¹⁴ but instead of grading symptom severity as 0-3, a visual analog scale (VAS) was used. It was anticipated that the use of this continuous variable (VAS) would provide greater sensitivity in differentiating between the two treatment groups than would be provided by a dichotomous outcome parameter, such as that employed in the previous study. Thus, the planned sample size of 30 per group was expected to provide adequate sensitivity to differentiate between the Lotemax® and the placebo groups with respect to both the subjective and objective outcome parameters.

### Results:

The demography of the patients who entered the study is shown in Table 3 below. A total of 66 patients were enrolled of which 32 received Lotemax and 34 received the vehicle of Lotemax. The majority were female (75.7%) and there was a slightly larger preponderance of female patients in the vehicle treated group (88.2%) versus the Lotemax treated group (62.5%). There were 4 patients who had a history of punctal occlusion and all of these had been randomly assigned to receive Lotemax.

**TABLE 3. Demographic Data**

| | | Lotemax® | Placebo | All Patients |
|---|---|---|---|---|
| Variable | | | | |
| | | n=32 | n=34 | n=66 |
| Mean age (years) | | 57.6 | 56.2 | 56.9 |
| Sex | Male | 12 (37.5%) | 4 (11.8%) | 16 (24.2%) |
| | Female | 20 (62.5%) | 30 (88.2%) | 50 (75.7%) |
| Race | Caucasian | 30 (93.8%) | 31 (91.2%) | 61 (92.4%) |
| | Black | 0 (0.0%) | 0 (0.0%) | 0 (0.0%) |
| | Hispanic | 2 (6.3%) | 2 (5.9%) | 4 (6.1%) |
| | Other | 0 (0.0%) | 1 (2.9%) | 1 (1.5%) |
| Iris | Brown | 10 (31.3%) | 12 (35.3%) | 22 (33.3%) |
| | Blue | 13 (40.6%) | 13 (38.2%) | 26 (39.4%) |
| | Hazel | 2 (6.3%) | 3 (8.8%) | 5 (7.6%) |
| | Green | 6 (18.8%) | 5 (14.7%) | 11 (16.7%) |
| | Other | 1 (3.1%) | 1 (2.9%) | 2 (3.0%) |
| Current or past medical | | | | |
| condition | | 29 (90.6%) | 31 (91.2%) | 60 (90.9%) |
| Previous surgical procedures | | 22 (68.8%) | 31 (91.2%) | 53 (80.3%) |
| Ocular | | 11 (34.4%) | 14 (41.2%) | 25 (37.9%) |
| Punctal occlusion | | 4 (12.5%) | 0 (0.0%) | 4 (6.1%) |

### Efficacy:

Efficacy was analyzed as the change in signs and symptoms from the Baseline provided by Visit 2. Two periods were analyzed for within-group and between-group differences: Visit 2 to Visit 3, which was the change during the first 2 weeks on study drug; and Visit 2 to Visit 4, which was the change during the whole 4 weeks on study drug.

In addition the change from Visit 4 to Visit 5 was analyzed since this was the immediate period after the study drug was stopped and would indicate a regression or loss of the treatment effect after withdrawal of the study drug. These results for the Primary Subjective Variable (worst symptom) and primary Objective Variable (Composite corneal staining score for the worst eye), the inferior tarsal conjunctiva injection score and the nasal bulbar conjunctival injection score are shown in Table 4 below.

**Table 4: Mean Change in Sign and Symptom Scores: All Patients Treated Analysis**

| | | Lotemax N=32 | | | Placebo/Vehicle N=34 | | | Between groups |
|---|---|---|---|---|---|---|---|---|
| Period | Variable | Mean | SD | p-value* | Mean | SD | p-value* | p-value** |
| Visit 2-3 | 1⁰ Subjective | -25.68 | 23.21 | <*0.0001* | -28.00 | 28.92 | <*0.0001* | 0.7253 |
| | 1⁰ Objective | -0.52 | 4.53 | 0.5307 | 0.00 | 3.63 | 1.0000 | 0.6158 |
| | Nasal Conj Hyperemia | -0.35 | 0.55 | *0.0012* | 0.00 | 0.43 | 1.0000 | *0.0055* |
| | Inf Tarsal Hyperemia | -0.29 | 0.59 | *0.0101* | 0.00 | 0.56 | 1.0000 | *0.0473* |
| Visit 2-4 | 1⁰ Subjective | -31.58 | 24.77 | <*0.0001* | -34.55 | 25.70 | <*0.0001* | 0.6404 |
| | 1⁰ Objective | -0.65 | 3.61 | 0.3278 | -0.36 | 3.85 | 0.5909 | 0.7641 |
| | Nasal Conj Hyperemia | -0.29 | 0.46 | *0.0015* | -0.06 | 0.43 | 0.4226 | *0.0431* |
| | Inf Tarsal Hyperemia | -0.19 | 0.65 | 0.1099 | -0.06 | 0.56 | 0.5354 | 0.3833 |
| Visit 4-5 | 1⁰ Subjective | 10.06 | 28.35 | 0.0573 | 11.36 | 25.93 | *0.0170* | 0.8488 |
| | 1⁰ Objective | 0.10 | 3.32 | 0.8722 | 0.24 | 2.72 | 0.6117 | 0.8479 |
| | Nasal Conj Hyperemia | 0.10 | 0.40 | 0.1840 | 0.03 | 0.03 | 0.5717 | 0.4530 |
| | Inf Tarsal Hyperemia | 0.03 | 0.55 | 0.7448 | 0.06 | 0.43 | 0.4226 | 0.8176 |

| | | | | | | | | |
|---|---|---|---|---|---|---|---|---|
| *Within group comparison (compared to zero). ** Between group comparison | | | | | | | | |

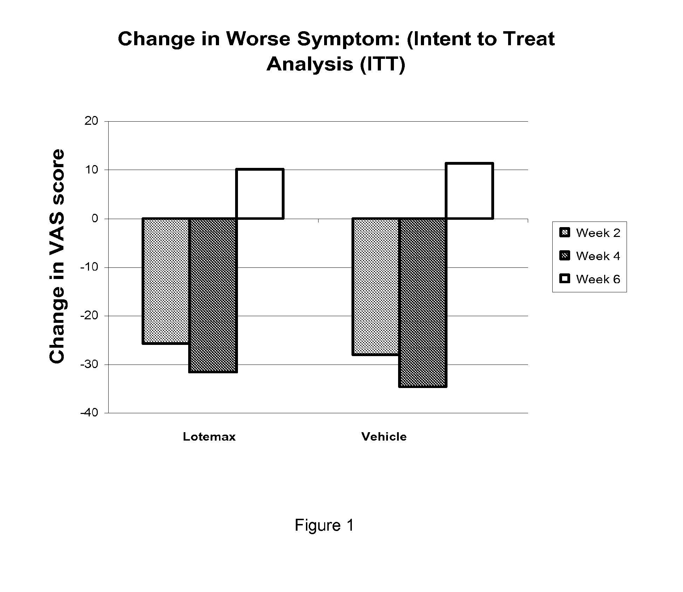

Table 4 shows that in the All Patients Treated analysis (Lotemax n=32, Vehicle n=34) a clear treatment effect was seen within both groups. There was a significant within treatment improvement in the worst symptom (Primary Subjective Variable) in both groups during the first 2 weeks and during the total 4 weeks of treatment. The regression after treatment was significant in the vehicle group but not in the Lotemax treatment group (Figure 1). Similar trends were seen for the combined corneal staining score (Primary Objective Variable) but to a much lesser extent.

In the nasal bulbar conjunctival injection (hyperemia) scores there was a statistically significant improvement during the first 2 weeks and during the 4 week treatment period as a whole in the patients treated with Lotemax, but not in the patients treated with vehicle. The difference between the treatment groups was statistically significant (p= 0.0431). There was more regression of effect between Visits 4 and 5 in the Lotemax treated group than in the vehicle treated group largely arising from the absence of any treatment effect in the vehicle treated group.

In the inferior tarsal conjunctival hyperemia scores there was again a significant improvement in the Lotemax treated group during the first 2 weeks which was significantly different from the vehicle treated group (p=0.0473). A similar pattern was seen in the 4 week treatment period, although this was not significant. After Visit 4 there was a similar regression in both the vehicle treatment group and in the Lotemax treated group.

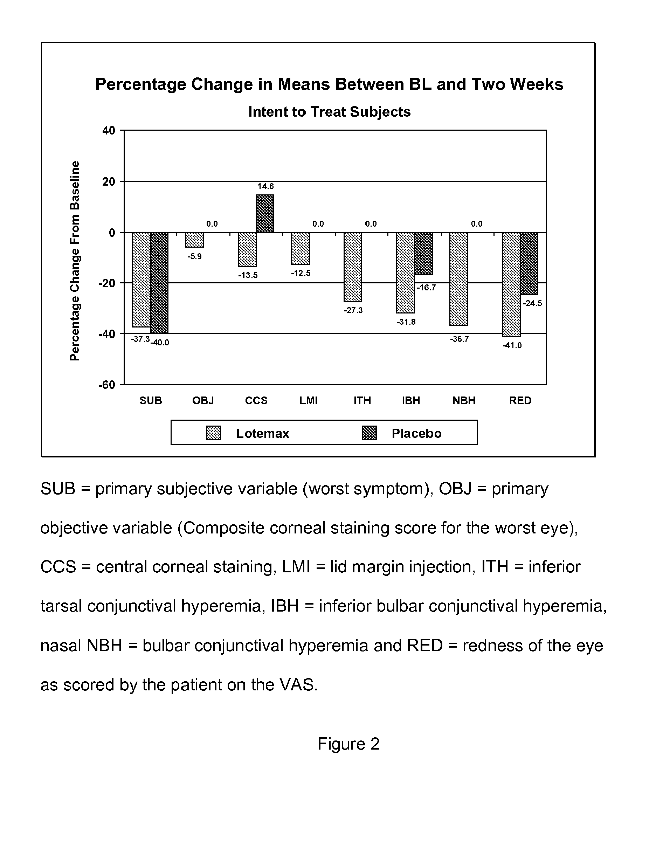

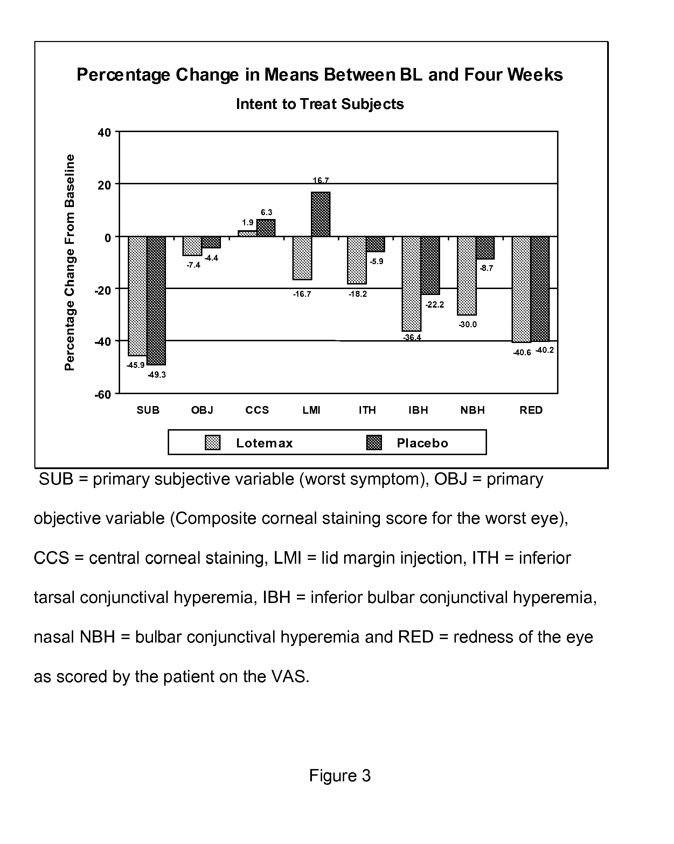

Figures 2 and 3 show for the ITT population, the percentage change from Baseline to week 2 and from Baseline to week 4, respectively, in the primary subjective (SUB) and objective (OBJ) variables, and also for central corneal staining (CCS), lid margin injection (LMI), inferior tarsal conjunctival hyperemia (ITH), inferior bulbar conjunctival hyperemia (IBH), nasal bulbar conjunctival hyperemia (NBH) and redness (RED) of the eye as scored by the patient on the VAS.

Analysis of the usage of Moisture Eyes artificial tear drops throughout the study showed no significant difference between the treatment groups at any of the visits.

At Visit 2 the mean Schirmer 1 test scores were Lotemax®: 9.16 + 7.65mm, and placebo: 11.18 + 9.70mm. At Visit 4 they were 10.03 + 7.91 mm and 10.91 + 5.73mm. There were no changes in the mean Schirmer 1 test scores that appeared to be related to treatment or treatment groups.

Although the majority of enrolled patients were female, the treatment effect (difference between the treatment groups) did not differ significantly between males and females.

Because this was the first clinical trial conducted to evaluate Lotemax for treatment of this condition, it was designed as a pilot study for future investigations. The patient population was chosen according to un-tested criteria which were selected based on clinical experience rather than on previous clinical trials of the use of steroid in dry eye disease. Therefore, the patients included in the study - whilst meeting some requirements for severity of signs and symptoms, as required by the protocol - still varied widely in the severity of their dry eye disease and in the amount of inflammation they had associated with their KCS. There were substantial subgroups of patients who had moderate to severe clinical inflammatory signs and symptoms, for whom the potential to benefit from the use of a steroid was clearly greater. These subgroups provided the greater therapeutic challenge for Lotemax and the efficacy analysis presented below is the evaluation of this moderate to severe subgroup. Data from all patients were included in the safety analysis.

### Efficacy subset analysis:

The following criteria were selected to define subpopulations of patients who had KCS with at least a moderate clinical inflammatory component: defined as a) a combined corneal staining score ≥ 10, b) conjunctival injection in at least one area of ≥ 2, c) a worst symptom score on VAS (Primary Subjective variable) ≥ 70, d) redness symptom score on VAS of ≥ 70. These subsets were labeled respectively a)Cst, b)Conj, c)Subj and d)Red. The subsets were analyzed alone and in combination. Table 5 below lists the subsets included in this efficacy analysis. For further analysis only the combined subsets in which one arm contained at least 10 patients were considered.

**TABLE 5. Characteristics of Efficacy Analysis Subsets**

| **Subset** | Description | Lotemax® | Vehicle |
|---|---|---|---|
| | | n | n |
| **Subj.** | | | |
| | Primary subjective variable | | |
| | | 19 | 23 |
| | (VAS worst symptom) =>70 | | |
| **Cst.** | Primary objective variable | | |
| | (composite corneal staining | 13 | 6 |
| | score) =>10 | | |
| **Red** | VAS redness score upon | | |
| | waking or during the day of | 10 | 16 |
| | =>70 | | |
| **Conj.** | | | |
| | Conjunctival injection score in | | |
| | | 14 | 13 |
| | any of the three areas of=>2 | | |
| **Subj. and** | VAS worst symptom | | |
| **Red⁸** | score=>70 and VAS redness | 9 | 14 |
| | score of=>70 | | |
| **Subj. and** | VAS worst symptom score=>70 | | |
| **Conj⁸** | and conjunctival injection in at | 10 | 9 |
| | least 1 area=>2 | | |
| **Cst. and** | Composite corneal staining | | |
| **Conj⁸** | score=>10 and conjunctival injection | 10 | 4 |
| | in at least 1 area=>2 | | |

| | | | |
|---|---|---|---|
| * Those patients who met both of the criteria for inclusion in the subset | | | |

The groups of patients who met at least two of the criteria for moderate to severe inflammatory KCS were analyzed for treatment effect (Table 6 below). A treatment effect was defined as the difference between active and placebo (Lotemax and Vehicle) either based on the mean scores or on the change in means between two timepoints. Visit 2, which was the end of the washout period, was used as the baseline for calculating change.

**Table 6: Difference between Lotemax and Vehicle - the difference in mean change from Visit 2 (baseline)¹. Subset analysis.**

| | Subgroup | Subjective (Worse symptom) | Redness During the Day | Central Corneal Staining² | Lid Margin Injection | Nasal Bulbar Conj | Inferior Tarsal Conj |
|---|---|---|---|---|---|---|---|
| Visits 2-3 | Subj/Red | 9.21 | -17.06 | **-*1.31**** | -0.18 | **-*0.78**** | -0.29 |
| | Subj/Conj | -6.5 | -21.04 | **-*0.9**** | 0.01 | **-*0.69**** | -0.29 |
| | Cst/Conj | -22.1 | -29.50 | -1.00 | 0.10 | -0.85* | -0.15 |
| Visits 2-4 | Subj/Red | 1.24 | -16.94 | -0.29 | **-*0.36**** | **-*0.67**** | -0.29 |
| | Subj/Conj | -6.48 | -13.84 | -0.29 | -0.2 | -0.38 | 0.12 |
| | Cst/Conj | -6.85 | -16.40 | -0.5 | 0.00 | -0.05 | 0.4 |

| | | | | | | | |
|---|---|---|---|---|---|---|---|
| ¹Obtained by subtracting the mean change for placebo from the mean change for Lotemax. A negative value indicates an improvement. ²Changes in Central corneal staining shown because of the statistical significant differences seen and because of its clinical relevance compared to other areas of the cornea. *= p <0.05 | | | | | | | |

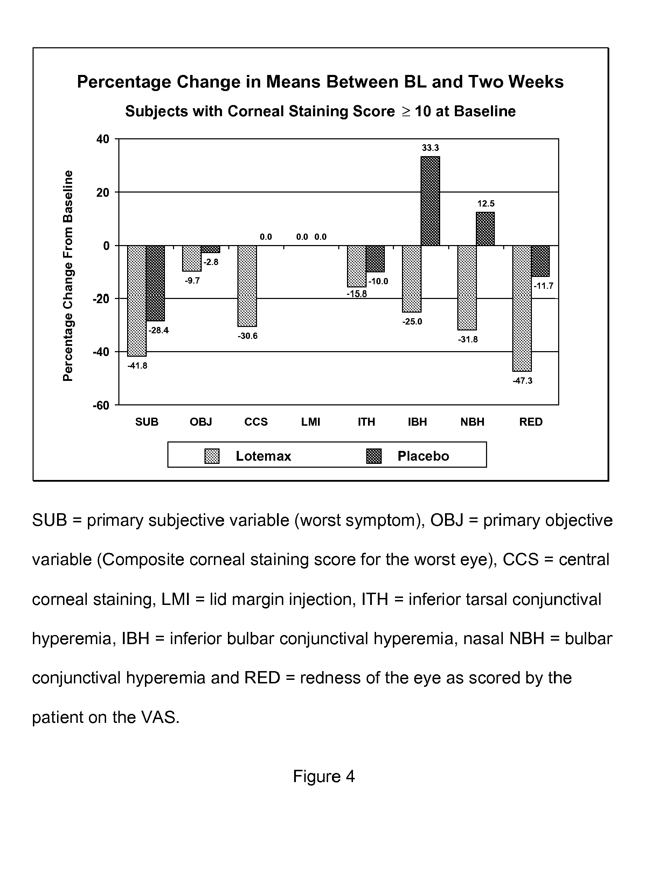

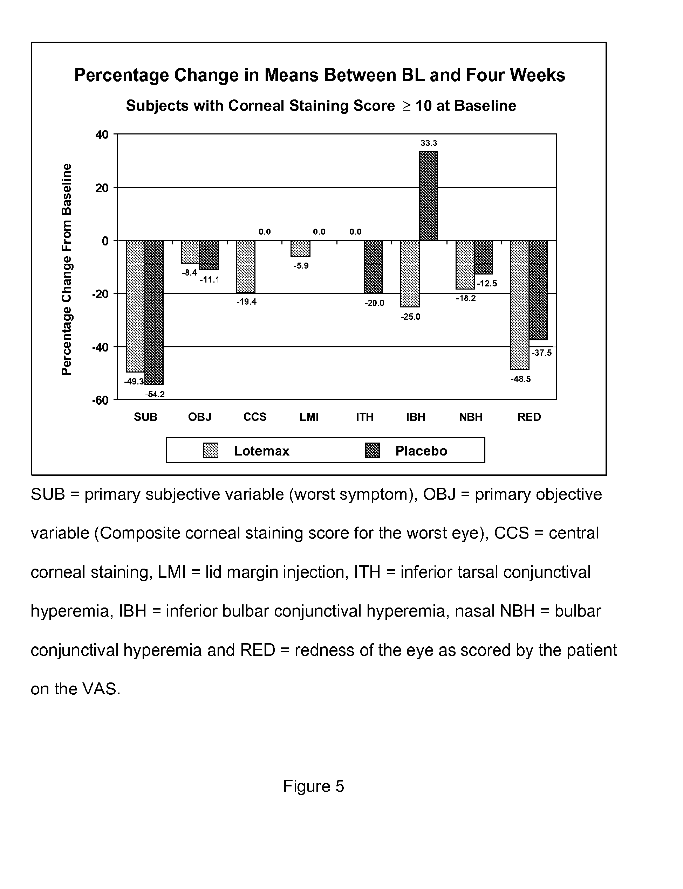

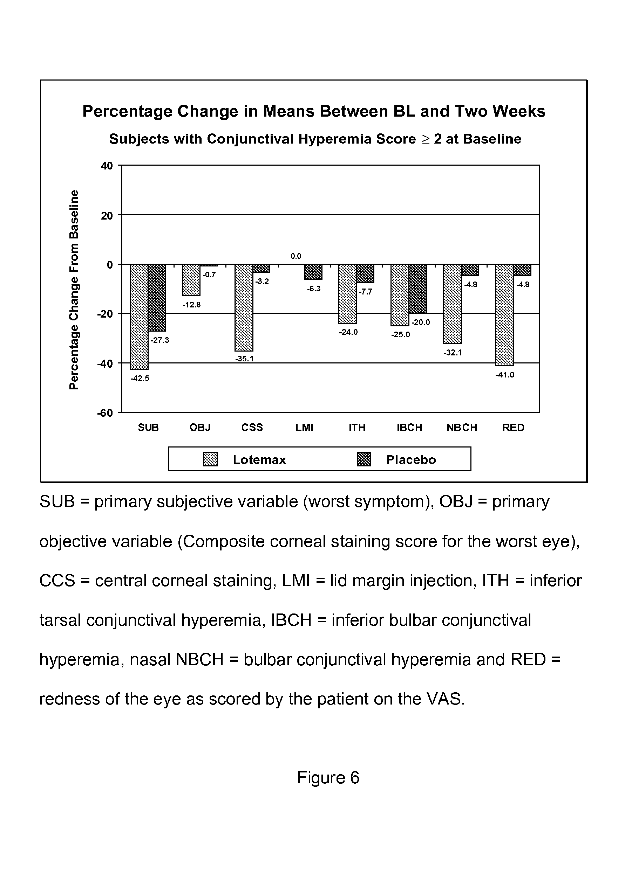

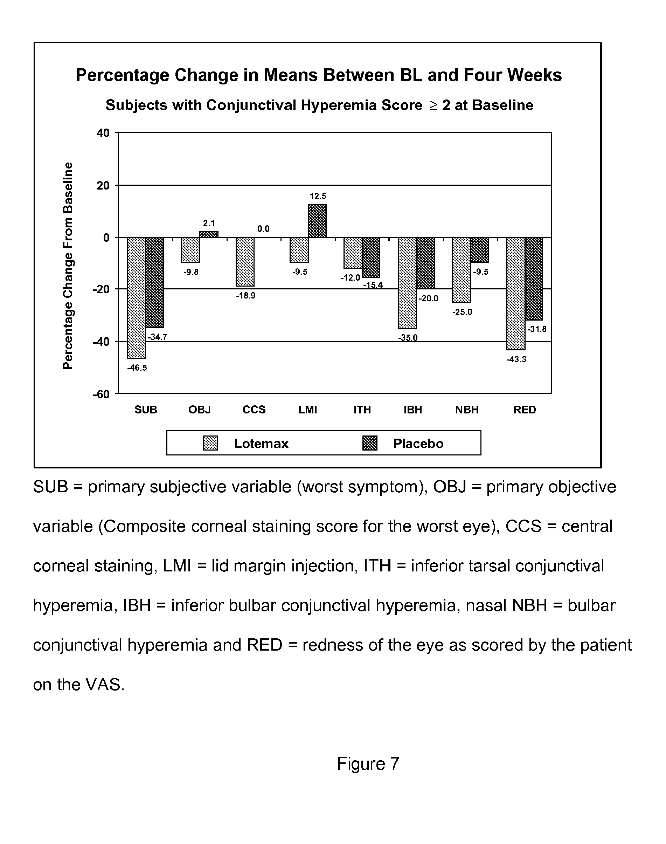

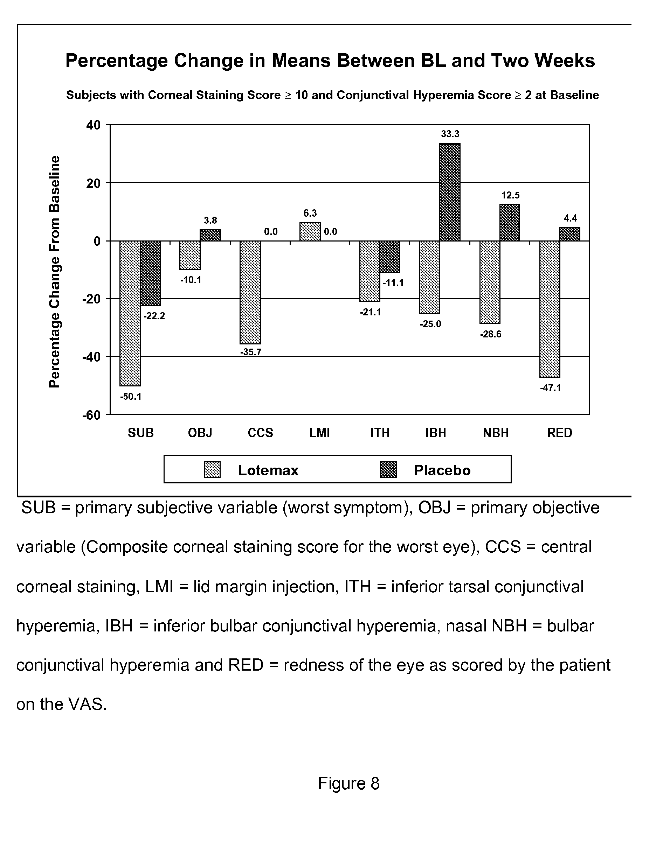

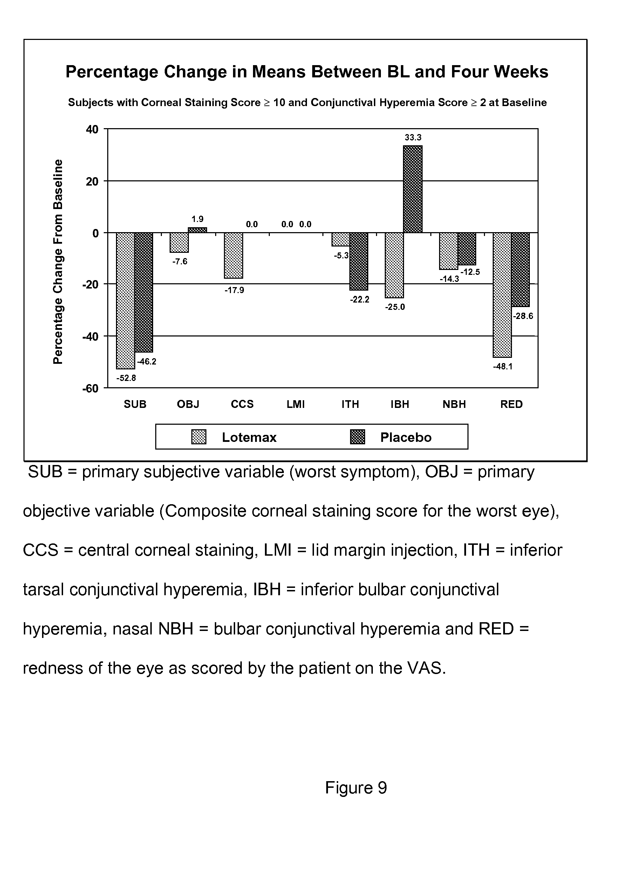

The percentage change from baseline after 2 weeks and 4 weeks on treatment is shown graphically for the patients with a combined corneal staining score of >10 at baseline (Figures 4 and 5), those with a conjunctival hyperemia score in one area at baseline of >2 (Figures 6 and 7) and the combined subset Cst and Conj, in Figures 8 and 9. The variables most likely to demonstrate an anti-inflammatory effect were included. The worst symptom at baseline (Primary Subjective Variable) was clearly of interest to show symptomatic improvement. Redness during the day was the patient's VAS score for the appearance of their eye. The other variables presented are: central corneal fluorescein staining, inferior tarsal hyperemia, nasal bulbar conjunctival hyperemia and lid margin injection.

The difference in the change in worse symptom score was greater in the "Subj and Conj" and in the "Cst and Conj" groups. The improvement in the redness VAS score was consistently around 20% better in the Lotemax treated patients compared to the vehicle treated patients. At the 2-week visit all subsets found a benefit from Lotemax of around 1 unit (or approximately 33% on a 0-3 grading scale) over vehicle in the central corneal staining score. This was significant in the Subj and Red and the Subj and Conj subsets during the first 2 weeks of treatment (Visit 2-3). The difference between the treatment groups was less after week 4 (Visit 4). The improvement in lid margin injection was statistically significant only in the Subj and Red subset during the period Visit 2-4 improvement was present in the period Visit 2-3 but to a lesser extent.

The improvement in nasal bulbar conjunctival injection compared to vehicle was just less than a 1 unit change (-0.69 to -0.85 or approximately 26%) in all three subsets after 2 weeks (Visit 3) and in the Subj and Red subsets after 4 weeks (Visit 4) were it reached statistical significance. There was also an improvement in inferior tarsal conjunctival injection, and in the inferior bulbar conjunctival injection (not shown).

### Videokeratoscopy

The surface regularity index (SRI) of the Tomey TMS-2N was evaluated before and after treatment in the worst eye of 8 subjects at one of the study sites. A significant decrease in the SRI was observed after one month of treatment with Lotemax®, while there was no difference between the pre- and post-treatment values in the vehicle treated patients (Table 7 below).

**Table 7: Surface Regularity Index (Mean ± SD)**

| | **Lotemax®** (n=4) | **Vehicle** (n=4) |
|---|---|---|
| **Pre-treatment** | 1.29 ± 0.48 | 1.04 ± 0.47 |
| **Post-treatment (4 weeks)** | 0.93 ± 0.5 | 1.09 ± 0.31 |
| | p < 0.04 | NS |

### Safety Analysis

There were 30 adverse events reported by 15 patients in the Lotemax® treated group (46.9%), and 25 adverse events reported by 17 patients in the placebo treated group (50.0%). Ocular adverse events accounted for 56.7% of the total adverse events reported by those treated with Lotemax®, and 56% of the total in the vehicle treated group. Of all adverse events, 16.7% and 23.5% were regarded as treatment related (Lotemax® and placebo, respectively). Four serious adverse events were reported none of which was treatment related, and two of these patients were discontinued from the study. Table 8 below lists treatment-related ocular adverse events.

**TABLE 8. Summary of Treatment-Related Ocular Adverse Events occurring in 2 or more patients.**

| | Lotemax® | Placebo |
|---|---|---|
| | n=32 | n= 34 |
| Adverse event | | |
| Increased burning | 2 | 4 |
| Redness | 3 | 1 |
| Blurring of vision¹ | 3 | 0 |
| Foreign body sensation | 1 | 3 |

| | | |
|---|---|---|
| ¹One patient put superglue in her eye accidentally | | |

No clinically significant change in visual acuity was found in any patient in either treatment arm. Slit lamp examination, lens examination, and fundoscopy performed at the end of the treatment period revealed no treatment related abnormalities. In particular there were no signs of cataract formation and there was no clinically significant change in IOP in any patient in the study. The mean IOP data are shown in Table 9 below.

**Table 9: Intra Ocular Pressure (mmHg): Means and Standard Deviations by visit.**

| | LOTEMAX® | | | Vehicle | | |
|---|---|---|---|---|---|---|
| | n | Mean | SD | n | Mean | SD |
| Visit 1 | 32 | 14.38 | 2.62 | 34 | 15.71 | 3.48 |
| Visit 2 | 30 | 14.23 | 2.69 | 32 | 15.59 | 3.03 |
| Visit 3 | 31 | 14.39 | 2.80 | 33 | 15.70 | 2.94 |
| Visit 4 | 31 | 15.29 | 3.28 | 33 | 14.97 | 3.50 |
| Visit 5 | 31 | 14.19 | 2.90 | 33 | 15.00 | 3.09 |

This invention has been described by reference to certain preferred embodiments; however, it should be understood that it may be embodied in other specific forms or variations thereof without departing from its special or essential characteristics. The embodiments described above are therefore considered to be illustrative in all respects and not restrictive, the scope of the invention being indicated by the appended claims rather than by the foregoing description.

The claims, as originally presented and as they may be amended, encompass variations, alternatives, modifications, improvements, equivalents, and substantial equivalents of the embodiments and teachings disclosed herein, including those that are presently unforeseen or unappreciated, and that, for example, may arise from applicants/patentees and others.

### REFERENCES

1. Pflugfelder SC. Anti-inflammatory therapy of dry eye. The Ocular Surface 2003; 1:31-36
2. Afonso AA, Sobrin L, Monroy DC, Selzer M, Lokeshwar B, Pflugfelder SC. Tear fluid gelatinase B activity correlates with IL-1 alpha concentration and fluorescein clearance in ocular rosacea. Invest Ophthalmol Vis Sci 1999;40:2506-12
3. Brignole F., Pisella P.J., Goldschild M., De Saint Jean M., Goguel A., Baudouin C. Flow cytometric analysis of inflammatory markers in conjunctival epithelial cells of patients with dry eyes. Invest Ophthalmol Vis Sci. 2000; 41: 1356-1363.
4. Stern ME, Gao J, Schwalb TA, Ngo M, Tieu DD, Chan CC, Reis BL, Whitcup SM, Thompson D, Smith JA. Conjunctival T-Cell Subpopulations in Sjogren's and Non-Sjogren's Patients with Dry Eye. Invest Ophthalmol Vis Sci 2002;43:2609-14
5. Solomon A, Dursun D, Liu Z, Xie Y, Macri A, Pflugfelder SC. Pro- and anti-inflammatory forms of interleukin-1 in the tear fluid and conjunctiva of patients with dry-eye disease. Invest Ophthalmol Vis Sci. 2001 ;42:2283-2292.
6. Pflugfelder SC, Jones D, Ji Z, Afonso A, Monroy D. Altered cytokine balance in the tear fluid and conjunctiva of patients with Sjogren's syndrome keratoconjunctivitis sicca. Curr Eye Res. 1999;19:201-211.
7. Tishler M, Yaron I, Geyer O, Shirazi I, Naftaliev E, Yaron M. Elevated tear interleukin-6 levels in patients with Sjogren syndrome. Ophthalmology. 1998;105:2327-2329.
8. Li DQ, Lokeshwar BL, Solomon A, Monroy D, Ji Z, Pflugfelder SC. Regulation of MMP-9 production by human corneal epithelial cells. Exp Eye Res 2001;73:449-59
9. Pflugfelder SC, Farley W, Li D-Q, Song X, Fini E. Matrix Metalloproteinase-9 (MMP-9) Knockout Alters the Ocular Surface Response to Experimental Dryness. Invest. Ophthalmol. Vis. Sci. 2002 43: E-Abstract 3124.
10. Solomon A, Monroy D, Rosenblatt M, Ji Z, Lokeshwar BL, Pflugfelder SC. Doxycycline inhibition of interleukin-1 in the corneal epithelium. Invest Ophthalmol Vis Sci 2000; 41:2544-57
11. Dursun D, Kim MC, Solomon A, Pflugfelder SC. Treatment of recalcitrant recurrent corneal epithelial erosions with inhibitors of matrix metalloproteinases-9, doxycyclne and corticosteroids. Am J Ophthalmol 2001; 132:8-13
12. Pflugfelder SC, Solomon A, Stern ME. The diagnosis and management of dry eye: a twenty-five-year review. Cornea 2000;19:644-9.
13. Marsh P, Pflugfelder SC. Topical nonpreserved methylprednisolone therapy for keratoconjunctivitis sicca in Sjogren's syndrome. Ophthalmology 1999;106:811-16.
14. Sainz de la Maza Serra M, Simon Castellvi C, Kabbani O. Nonpreserved topical steroids and punctal occlusion for severe keratoconjunctivitis sicca [in Spanish]. Arch Soc Esp Oftalmol 2000;75:751.6.
15. Macri A, Rolando M, Pflugfelder SC. A standardized visual scale for evaluation of tear fluorescein clearance. Ophthalmology 2000;107:1338 -43.
16. de Paiva CS, Pflugfelder SC. Assessing the severity of keratitis sicca with videokeratoscopic indices, Ophthalmology 2003; 110:1102-9.
17. McCarty CA, Bansal AK, Livingston PM, Stanislavsky YL, Taylor HR. The epidemiology of dry eye in Melbourne, Australia. Ophthalmology. 1998; 105: 1114-1119.
18. Moss SE, Klein R, Klein BE. Prevalence of and risk factors for dry eye syndrome. Arch Ophthalmol. 2000; 118: 1264-1268.
19. Prabhasawat P, Tseng SCG. Frequent association of delayed tear clearance in ocular irritation Br J Ophthalmol 1998;82:666-75
20. Ilyas H, Slonim CB, Braswell GR, Favetta JR, Schulman M. Long-term safety of loteprednol etabonate 0.2% in the treatment of seasonal and perennial allergic conjunctivitis. Eye & Contact Lens 2004;30:10-13.
21. Ichijima H, Petrol WM, Jester JV, Cavanaugh HD: Confocal microscopic studies of living rabbit cornea treated with benzalkonium chloride, Cornea 11:221-225,1992.
22. Pfister RR, Burstein N: The effects of ophthalmic drugs, vehicles, and preservatives on corneal epithelium: a scanning electron microscopic study, Invest Ophthalmol Vis Sci 1976;15:246-259.

In view of the above, some aspects of the invention relate to:
1. A method for the treatment of moderate to severe dry eye comprising:
   administering to a mammal a formulation comprising a pharmaceutically acceptable carrier and a moderate to severe dry eye treatment effective amount of (11β,17α),-17-[(Ethoxycarbonyl)oxy]-11-hydroxy-3-oxoandrosta-1,4-diene-17-carboxylic acid chloromethyl ester.
2. The method of aspect 1 wherein the pharmaceutically effective amount of (11β,17α),-17-[(Ethoxycarbonyl)oxy]-11-hydroxy-3-oxoandrosta-1,4-diene-17-carboxylic acid chloromethyl ester is between 0.001-5.0% (W/W).
3. The method of aspect 2 wherein the pharmaceutically effective amount of (11β,17α),-17-[(Ethoxycarbonyl)oxy]-11-hydroxy-3-oxoandrosta-1,4-diene-17-carboxylic acid chloromethyl ester is between 0.001-1.0% (W/W).
4. The method of aspect 1 wherein the formulation is topically administered to the eye.
5. The method of aspect 1 wherein the moderate to severe dry eye is associated with refractive surgery.
6. The method of aspect 1 wherein the formulation is an ophthalmic suspension.
7. The method of aspect 6 wherein the ophthalmic suspension comprises 0.5 wt percent of Loteprednol etabonate in a pharmaceutical carrier comprising povidone, benzalkonium chloride, disodium EDTA, glycerin, tyloxapol and water.
8. The method of aspect 1 wherein the formulation is a gel.
9. The method of aspect 8 wherein the gel compromises acrylic acid-based polymer, water, propylene glycol, EDTA and Loteprednol etabonate.
10. The method of aspect 9 wherein the gel further compromises triacetin.
11. The method of aspect 8 wherein the formulation compromises acrylic acid-based polymer, water, propylene glycol, glycerin, EDTA, benzalkonium chloride and Loteprednol etabonate.
12. An ophthalmic gel formulation suitable for the treatment of moderate to severe dry eye comprising Loteprednol etabonate.
13. The formulation of aspect 11 further comprising at least one member selected from the group consisting of water, acrylic-based polymers, propylene glycol, EDTA, triacetin and benzalkonium chloride.
14. A kit for the treatment of moderate to severe dry eye, the kit comprising:
   a pharmaceutical formulation comprising Loteprednol etabonate contained in a pharmaceutically acceptable container;
   a written package insert containing instructions for using the formulation for the treatment of moderate to severe dry eye; and
   outer packaging identifying the pharmaceutical formulation contained therein.
15. The kit of aspect 13 wherein the pharmaceutically acceptable container is suitable for single use by a user of the formulation contained in the package.
16. The kit of aspect 13 wherein the outer packaging contains at least one pharmaceutically acceptable container containing the Loteprednol etabonate pharmaceutical formulation.
17. A method for the treatment of chronic dry eye, the method comprising:
   administering to a patient in need of treatment thereof a treatment formulation comprising a therapeutic amount of Loteprednol etabonate in a pharmaceutically acceptable carrier; and
   continuing to administer the treatment formulation for a period of greater than one month.
18. The method of aspect 16 wherein the treatment is administered for a period of greater than three years.
19. The method of aspect 16 wherein the treatment formulation is preservative free.
20. The method of aspect 18 wherein the treatment formulation is preservative free.
21. A method for reducing conjunctival redness associated with dry eye, the method comprising:
   administering to a patient in need of treatment thereof a treatment formulation comprising a therapeutic amount of Loteprednol etabonate in a pharmaceutically acceptable carrier.
22. The method of aspect 21 further comprising the step of continuing to administer the treatment formulation for a period of greater than one month.
23. The method of aspect 22 wherein the treatment is administered for a period of greater than three years.
24. A method for decreasing the production of inflammatory cytokines by cells in the epithelium, the method comprising:
   administering to a patient in need of treatment thereof a treatment formulation comprising a therapeutic amount of Loteprednol etabonate in a pharmaceutically acceptable carrier.
25. The method of aspect 23 further comprising the step of continuing to administer the treatment formulation for a period of greater than one month.
26. The method of aspect 24 further comprising the step of continuing to administer the treatment formulation for a period of greater than one month.
27. The method of aspect 25 wherein the treatment is administered for a period of greater than three years.

## Claims

1. Use of (11β, 17α),-17-[(Ethoxycarbonyl)oxy]-11-hydroxy-3-oxoandrosta-1,4-diene-17-carboxylic acid chloromethyl ester for the manufacture of a medicament for the treatment of chronic dry eye, wherein a formulation comprising a pharmaceutically acceptable carrier and an effective amount of (11β, 17α),-17-[(Ethoxycarbonyl)oxy]-11-hydroxy-3-oxoandrosta-1,4-diene-17-carboxylic acid chloromethyl ester is administered.

2. The use of claim 1, wherein the pharmaceutically effective amount of (11β, 17α),-17-[(Ethoxycarbonyl)oxy]-11-hydroxy-3-oxoandrosta-1,4-diene-17-carboxylic acid chloromethyl ester is between 0.001-5.0% (W/W).

3. The use of claim 2, wherein the pharmaceutically effective amount of (11β, 17α),-17-[(Ethoxycarbonyl)oxy]-11-hydroxy-3-oxoandrosta-1,4-diene-17-carboxylic acid chloromethyl ester is between 0.001-1.0% (W/W).

4. The use of claim 1, wherein the formulation is topically administered to the eye.

5. The use of claim 1, wherein the formulation is an ophthalmic suspension.

6. The use of claim 5, wherein the ophthalmic suspension comprises 0.5 wt. % of (11β, 17α),-17-[(Ethoxycarbonyl)oxy]-11-hydroxy-3-oxoandrosta-1,4-diene-17-carboxylic acid chloromethyl ester in a pharmaceutical carrier comprising povidone, benzalkonium chloride, disodium EDTA, glycerin, tyloxapol and water.

7. The use of claim 1, wherein the formulation is a gel.

8. The use of claim 7, wherein the gel comprises acrylic acid-based polymer, water, propylene, glycol, EDTA and (11β, 17α),-17-[(Ethoxycarbonyl)oxy]-11-hydroxy-3-oxoandrosta-1,4-diene-17-carboxylic acid chloromethyl ester.

9. The use of claim 8, wherein the gel further comprises triacetin.

10. The method of claim 7, wherein the formulation comprises acrylic acid-based polymer, water, propylene glycol, glycerin, EDTA, benzalkonium chloride and (11β, 17α),-17-[(Ethoxycarbonyl)oxy]-11-hydroxy-3-oxoandrosta-1,4-diene-17-carboxylic acid chloromethyl ester.

11. The use of claim 1, wherein the treatment is administered for a period of greater than one month.

12. The use of claim 11, wherein the treatment is administered for a period of greater than three years.

13. The use of claim 11, wherein the treatment formulation is preservative-free.

14. Use of (11β, 17α),-17-[(Ethoxycarbonyl)oxy]-11-hydroxy-3-oxoandrosta-1,4-diene-17-carboxylic acid chloromethyl ester for the manufacture of a medicament for reducing conjunctival redness associated with dry eye, wherein a formulation comprising a therapeutic amount of (11β, 17α),-17-[(Ethoxycarbonyl)oxy]-11-hydroxy-3-oxoandrosta-1,4-diene-17-carboxylic acid chloromethyl ester in a pharmaceutically acceptable carrier is administered.

15. The use of claim 14, wherein the treatment is administered for a period of greater than one month.

16. The use of claim 15, wherein the treatment is administered for a period of greater than three years.

17. Use of (11β, 17α),-17-[(Ethoxycarbonyl)oxy]-11-hydroxy-3-oxoandrosta-1,4-diene-17-carboxylic acid chloromethyl ester for the manufacture of a medicament for decreasing the production of inflammatory cytokines by cells in the epithelium, wherein the medicament comprises (11β, 17α),-17-[(Ethoxycarbonyl)oxy]-11-hydroxy-3-oxoandrosta-1,4-diene-17-carboxylic acid chloromethyl ester in a therapeutic amount in a pharmaceutically acceptable carrier.
